# EUROPEAN PATENT APPLICATION

(11) **EP 4 625 242 A1**
(43) Date of publication of application: **01.10.2025**
(21) Application number: 25162126.4
(22) Date of filing: 06.03.2025
(51) Int. Cl.: G06F 40/30, G06F 40/56

(54) **INFORMATION PROCESSING SYSTEM, INFORMATION PROCESSING METHOD, AND NON-TRANSITORY STORAGE MEDIUM**

(30) Priority: 27.03.2024 JP 2024050945
(71) Applicant: Canon Kabushiki Kaisha, Tokyo 146-8501 (JP)
(72) Inventor: UEDA, Ryuta, Tokyo, 146-8501 (JP); UENO, Shunya, Tokyo, 146-8501 (JP)
(74) Representative: Canon Europe Limited

(57) **Abstract**

An information processing system described in the present invention includes an input information acquisition unit (103a), an output information acquisition unit (103b), an identification unit (103c), and a display control unit (103d). The input information acquisition unit (103a) is configured to acquire input information for inputting into a sentence generation model. The output information acquisition unit (103b) is configured to acquire output information resulting from inputting the input information into the sentence generation model. The identification unit (103c) is configured to identify sections of the output information where user checking is recommended. The display control unit (103d) is configured to cause a display to display the output information in correspondence with the sections where user checking is recommended.

## Description

### BACKGROUND OF THE INVENTION

### Field of the Invention

The disclosure of the present specification relates to an information processing system, an information processing method, and a non-transitory storage medium.

### Description of the Related Art

Recent investigations are considering various ways of using sentences generated by sentence generation models, which are models that use artificial intelligence to generate sentences.

OpenAI, "GPT-4 Technical Report", arXiv:2303.08774v3, 2023 discloses ChatGPT, an interactive interface capable of using a sentence generation model referred to as a generative pre-trained transformer (GPT) to perform sentence-related tasks such as summarization, proofreading, and sentence generation with high accuracy.

### SUMMARY OF THE INVENTION

Sentence generation models such as ChatGPT are known to produce output composed of a large number of sentences and the like in response to text or other input information. However, the output information from sentence generation models may contain errors and the like due to hallucinations, making it necessary to check the output information, but checking all of the output information is laborious.

The present invention in its first aspect provides an information processing system as specified in claim 1. Optional features may be specified in claims 2-18.

The present invention in its second aspect provides an information processing method as specified in claim 19. Optional features may be specified in or derivable from claims 2 to 18.

The present invention in its third aspect provides a non-transitory storage medium as specified in claim 20. Optional features may be specified in or derivable from claims 2 to 18.

The present invention in its fourth aspect provides an information processing system comprising an input information acquisition unit that acquires input information for inputting into a sentence generation model and a constraint for causing the sentence generation model to output newly created information in a distinguishable manner from the input information; an output information acquisition unit that acquires output information resulting from inputting the input information into the sentence generation model; an identification unit that identifies sections of the output information where user checking is recommended; and a display control unit that causes a display to display the output information in correspondence with the sections where user checking is recommended.

The input information acquisition unit may further acquire, as the input information, information on an examination result concerning the subject. The input information acquisition unit may further acquire, as the input information, a result inferred by using an inference model on the information on an examination result concerning the subject. The information on an examination result concerning the subject may be information including medical image data acquired by examination of the subject. The information on an examination result concerning the subject may include a measured value acquired by examination of the subject.

The information acquisition unit may acquire the constraint for controlling the output of the sentence generation model so as to only add newly created information. The identification unit may identify differences between the input information and the output information as the sections where user checking is recommended.

The present invention in its fifth aspect may provide an information processing method comprising: acquiring input information for inputting into a sentence generation model and a constraint for causing the sentence generation model to output newly created information in a distinguishable manner from the input information; acquiring output information resulting from inputting the input information into the sentence generation model; identifying sections of the output information where user checking is recommended; and causing a display to display the output information in correspondence with the sections where user checking is recommended.

Further features of the present invention will become apparent from the following description of embodiments with reference to the attached drawings.

### BRIEF DESCRIPTION OF THE DRAWINGS

Fig. 1 is a diagram illustrating an example of the functional configuration of an information processing system according to a first embodiment.
Fig. 2 is a flowchart illustrating the flow of an example of processing by the information processing system according to the first embodiment.
Fig. 3 is a diagram illustrating an example of a diagnostic report entered by a user according to the first embodiment.
Fig. 4 is a diagram illustrating an example of a medical image (chest X-ray image) according to the first embodiment.
Fig. 5 is a diagram illustrating an example of an outputted diagnostic report according to the first embodiment.
Fig. 6 is a diagram illustrating an example of a display according to the first embodiment in which an outputted diagnostic report and sections where checking is recommended are associated with one another.
Fig. 7 is a flowchart illustrating the flow of an example of processing by an information processing system according to a second embodiment.
Fig. 8 is a diagram illustrating an example of an outputted diagnostic report according to the second embodiment.
Fig. 9 is a diagram illustrating an example of a display according to the second embodiment in which an outputted diagnostic report and sections where checking is recommended are associated with one another.

### DESCRIPTION OF THE EMBODIMENTS

The following describes embodiments of the present invention with reference to the drawings. Embodiments and modifications of an information processing system will be described in detail, with reference to the drawings. Note that an embodiment can be combined with related art, another embodiment, or a modification insofar as the content is non-contradictory. Similarly, a modification can be combined with related art, an embodiment, or another modification insofar as the content is non-contradictory. In the following description, similar component elements are denoted with the same signs, and redundant description may be omitted. The configuration of the information processing system is not limited to the embodiments described below, and may also be formed from multiple information processing devices that each include a unit, or may be formed from a smaller number of information processing devices that each include multiple units.

### FIRST EMBODIMENT

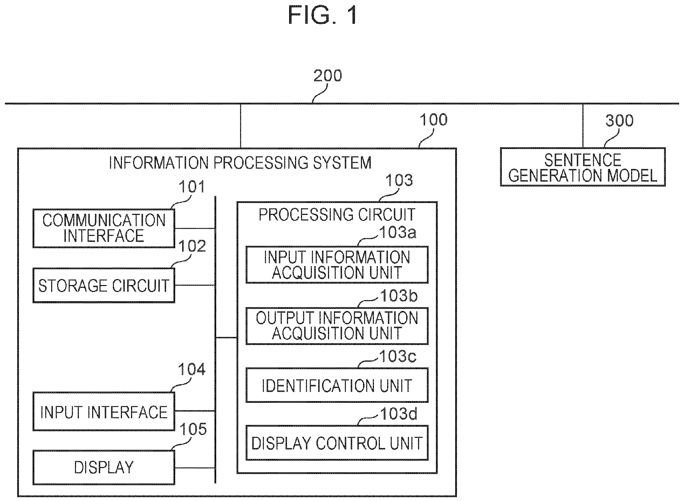

Fig. 1 is a diagram illustrating an example of the configuration of an information processing system 100 according to a first embodiment.

The information processing system 100 is a system configured to connect to a sentence generation model 300, typically a large language model such as a GPT, over a network 200. The present embodiment describes a configuration in which the information processing system 100 cooperates with an external sentence generation model 300 to acquire information outputted by the sentence generation model 300, but the sentence generation model 300 may also be realized as a component of the information processing system 100.

The information processing system 100 may also have at least one of the following functions: displaying an information processing result, saving the information processing result in association with text data and/or image data; outputting a result of information processing to an external device, and the like. In this context, input information is a diagnostic report concerning a subject, for example. The information processing system 100 according to the present embodiment identifies sections where user checking is recommended by comparing output information, such as a diagnostic report, outputted by the sentence generation model 300 with input information acquired by the information processing system 100. The information processing system 100 also carries out display control causing a display or other display unit to display sections where user checking is recommended, in correspondence with the output information from the sentence generation model 300. The information processing system 100 furthermore updates the diagnostic report on the basis of user-provided input regarding the sections where user checking is recommended, which are displayed on the display. In other words, the information processing system 100 generates a final diagnostic report in which the user has selected which sections to adopt or discard. The information processing system 100 may also cause the generated diagnostic report to be displayed on a connected display or a display device not illustrated. The information processing system 100 is realized by computer equipment such as a server or a workstation, for example.

Note that the information processing system 100 may also be communicatively connected to a data management device, not illustrated, in order to acquire a diagnostic report to be processed and/or save the final diagnostic report. The communication is executed over the network 200, for example, or via a communication cable, communication circuit, or the like not illustrated. The data management device stores various data, such as text data and medical image data of diagnostic reports, and is capable of transmitting and receiving various data to and from other devices, such as the information processing system 100 that can communicate with the data management device. Note that the data management device may also be integrated with the information processing system 100 as one component forming the information processing system 100.

The image data for inputting into the sentence generation model 300 has no particular limitations with respect to dimensionality or image type, but to give examples, the image data may be any of the following: three-dimensional image data (that is, volume data), for example medical image data taken by a device such as an optical coherence tomography (OCT) imaging device, a computed tomography (CT) device, a diagnostic ultrasound device, a magnetic resonance imaging (MRI) device, a positron emission computed tomography (PET) device, and/or a single-photon emission computed tomography (SPECT) device; two-dimensional image data, for example medical image data taken by a simple X-ray machine, a digital microphone, a fundus camera, and/or the like; and slice image data of the three-dimensional image data. As another example, the image data may also be three-dimensional data of a person, an animal, or an artifact acquired by a 3D scanner. The image data may also be three-dimensional image data generated by layering two-dimensional image data. Note that such image data may also be multidimensional image data with coordinate axes in four or more dimensions.

The following uses Fig. 1 to describes the functional configuration of the information processing system 100. The information processing system 100 includes a communication interface 101, a storage circuit 102, and a processing circuit 103. Depending on the circumstances in which the information processing system 100 is to be used, the information processing system 100 may also be provided with an input interface 104 and a display 105.

In the present embodiment, the information processing system 100 is communicatively connected to the network 200 via the communication interface 101.

The information processing system 100 communicates with the sentence generation model 300 over the network 200 to transmit information for inputting into the sentence generation model 300 and receive information outputted by the sentence generation model 300. Note that the sentence generation model 300 may also be integrated with the information processing system 100 as one component forming the information processing system 100.

The communication interface 101 is an interface for communicating diagnostic reports, medical image data, processing results, and the like with other devices. The communication interface 101 is realized by a network communication interface such as a network adapter or a network interface controller (NIC), or by a device connection interface such as Universal Serial Bus (USB), PCI Express, Serial ATA (SATA), or M.2, for example.

The storage circuit 102 stores various data and various programs. Specifically, the storage circuit 102 is connected to the processing circuit 103, and stores various data under control by the processing circuit 103. For example, the storage circuit 102 stores diagnostic reports and medical images under control by the processing circuit 103. The various data stored in the storage circuit 102 is read out and used by the processing circuit 103. The storage circuit 102 also functions as working memory for temporarily storing various data used in processing executed by the processing circuit 103. The storage circuit 102 is realized by a semiconductor memory element such as random-access memory (RAM) or flash memory, a hard disk, and/or an optical disc, for example.

The processing circuit 103 controls the information processing system 100 as a whole. For example, the processing circuit 103 performs various processing in response to a processing start instruction operation accepted from the user via the input interface 104 connected to the information processing system 100. As another example, the processing circuit 103 may also perform various processing in response to an analysis instruction operation accepted from the user via the communication interface 101. As another example, the processing circuit 103 may also perform various processing upon detecting that a diagnostic report has been stored in the storage circuit 102. The processing circuit 103 is realized by a processor, for example.

The processing circuit 103 in the information processing system 100 includes an input information acquisition unit 103a, an output information acquisition unit 103b, an identification unit 103c, and a display control unit 103d.

For example, the processing units which are component elements of the processing circuit 103 illustrated in Fig. 1 are stored in the storage circuit 102 in the form of computer-executable programs. The processing units are function units that include the input information acquisition unit 103a, the output information acquisition unit 103b, the identification unit 103c, and the display control unit 103d.

The processing circuit 103 realizes the functions corresponding to the programs by causing a processor to read out the programs from the storage circuit 102 and execute the read-out programs. In other words, the processing circuit 103, having read out the programs, functions as each of the units illustrated within the processing circuit 103 in Fig. 1.

The information processing system 100 includes the input information acquisition unit 103a that inputs input information into the sentence generation model 300, and the output information acquisition unit 103b that acquires output information obtained as a result of inputting input information into the sentence generation model 300. The information processing system 100 also includes the identification unit 103c that compares the input information with the output information to identify sections of the output information where user checking is recommended. The information processing system 100 further includes the display control unit 103d that causes the display 105 or other display unit to display the output information in correspondence with the sections where user checking is recommended. By being configured in this way, the present information processing system can present sections of the output information where user checking may be required, thus alleviating the user burden of checking the entire text of the output information.

The input interface 104 accepts input operations for various instructions and various information from the user of the information processing system 100. Specifically, the input interface 104 is connected to the processing circuit 103, converts input operations received from the user into electrical signals, and transmits the electrical signals to the processing circuit 103. For example, the input interface 104 is realized by a trackball, switches and/or buttons, a mouse, a keyboard, a touchpad with which input operations are performed by touching an operating surface, or the like. The input interface 104 may also be realized by a touchscreen combining a display screen with touchpad, a contactless input interface using an optical sensor, a voice input interface, and/or the like. Note that the input interface 104 is not limited to interfaces provided with physical operating parts such as a mouse and keyboard. For instance, examples of the input interface 104 also include an electrical signal processing circuit that receives an electrical signal corresponding to an input operation from an external input device provided separately from the information processing system 100, and transmits the electrical signal to the processing circuit 103.

The display 105 is an example of a display unit that displays various data, such as data based on a diagnostic report and/or processing result. Specifically, the display 105 is connected to the processing circuit 103, and displays various data received from the processing circuit 103, specifically the display control unit 103d. For example, the display 105 displays diagnostic reports based on text data and medical image data based on image data. The display 105 is realized by a liquid crystal display (LCD) monitor, a cathode ray tube (CRT) monitor, or a touch panel, for example.

The sentence generation model 300 is a system equipped with a large language model realized by machine learning technology such as a transformer model for generating sentences from inputted information. For example, the sentence generation model 300 outputs output information in response to input information such as a diagnostic report inputted from the processing circuit 103. The output information is the result of revising sentences and supplementing additional information with respect to a diagnostic report serving as input information. The sentence generation model 300 may also be a multimodal model, and may accept the input of medical image data and the like as input information, for example. Sentences may also be generated by inputting, as input information into the sentence generation model 300, examination results concerning a subject, such as medical image data and/or blood test data.

The sentence generation model 300 may also output a diagnostic report with added information about, for example, the location and size of a mass detected from a chest X-ray image and the presence or absence of inflammation estimated from CRP levels in blood tests. Note that the sentence generation model 300 may also be integrated with the information processing system 100 as one component forming the information processing system 100. In this case, the sentence generation model 300 may be stored in the storage circuit 102, and may be read out and used by the processing circuit 103. The sentence generation model 300 is realized by ChatGPT or Bidirectional Encoder Representations from Transformers (BERT), for example.

The foregoing describes a configuration example of the information processing system 100 according to the present embodiment. In the present embodiment, the information processing system 100 executes the various processing described below to create reports using a sentence generation model while alleviating the user burden of checking. The following describes an example of a procedure by which the information processing system 100 executes various processing for a diagnostic report.

The following describes an example of a report creation process executed by the information processing system 100 according to the present embodiment on a diagnostic report entered by the user, using ChatGPT as the sentence generation model 300. Note that processing similar to the present embodiment can be easily extended to text data other than diagnostic reports, and such other text data may be substituted into the following description, as necessary.

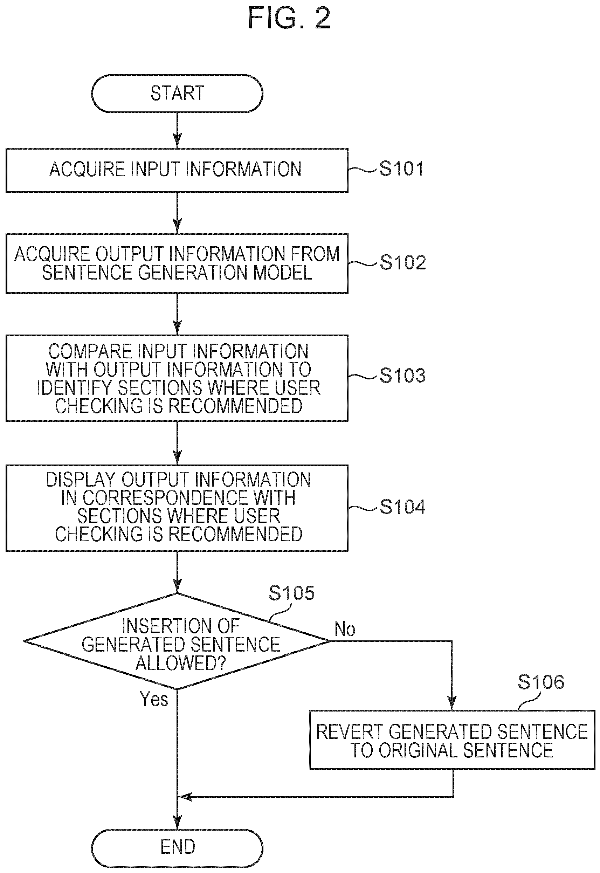

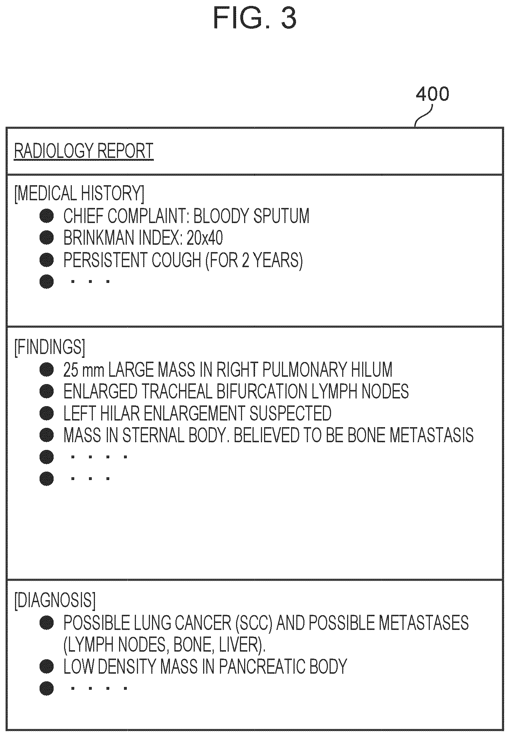

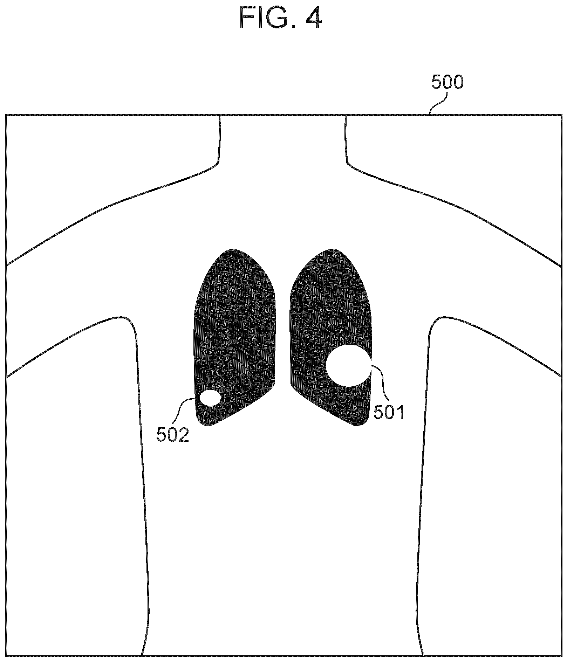

Fig. 2 is a flowchart illustrating the flow of an example of processing executed by the information processing system 100 according to the first embodiment. In the description of the present embodiment, execution of the processing illustrated in Fig. 2 is triggered when data to be processed is stored in the storage circuit 102 and the user operates the input interface 104 to give an instruction to start the processing. The data to be processed is, for example, text data of a diagnostic report 400 as illustrated in Fig. 3 and image data of a chest X-ray image 500 as illustrated in Fig. 4. The following description follows the steps of the flowchart illustrated in Fig. 2. Note that the steps may be reordered insofar as the content is non-contradictory, and certain steps may also be skipped.

In step S101, the input information acquisition unit 103a acquires input information for inputting into the sentence generation model 300. For example, the diagnostic report 400 and the chest X-ray image 500 as an examination result are acquired as the input information, and the process advances to the next step. Note that the information to be acquired as the input information may also be sentence information only.

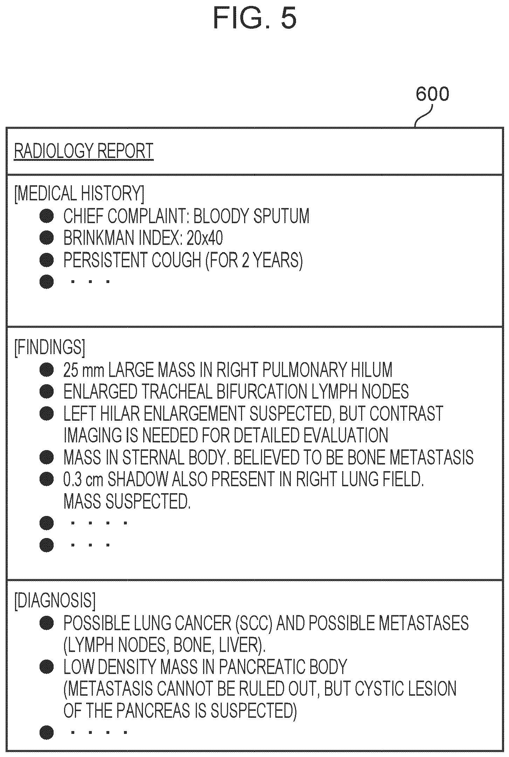

In step S102, the output information acquisition unit 103b transmits the diagnostic report 400 and the chest X-ray image 500 to the sentence generation model 300, and when output information to serve as an output result is acquired, the process advances to the next step. The output information is, for example, a diagnostic report 600 as illustrated in Fig. 5.

The diagnostic report serving as the output information includes, for example, added information about left hilar enlargement described in the diagnostic report 400 serving as the input information, the added information being obtained by an inference such as "contrast imaging is needed for detailed evaluation".

As another example, the diagnostic report includes added "information about a shadow 502 in the right lung field" not described in the diagnostic report 400 serving as the input information, the added information being obtained as a result of the sentence generation model making an inference based on the chest X-ray image 500, which is medical image data, serving as the input information. The output information acquisition unit 103b may additionally cause the sentence generation model 300 to output sentences added on the basis of the above inferences, and the likelihood of the above inferences. Alternatively, the input information acquisition unit 103a may add a constraint requiring likelihood output as part of the input information. That is, the input information acquisition unit 103a may acquire input information containing a constraint for inputting into the sentence generation model 300.

In step S103, the identification unit 103c compares the input information with the output information to identify sections of the output information where user checking is recommended, and then the process advances to the next step.
For example, the input information and the output information are converted into vectors, and similarity comparisons are made between individual sentence vectors. Note that this method of similarity comparison is an example, and differences between the input information and the output information may also be calculated according to an existing document comparison method. The identification unit 103c may also identify recommended sections on the basis of differences obtained by inputting the input information, the output information, and a prompt asking for output of the differences between the input and output information as input information into the sentence generation model 300. Alternatively, differences may be identified by inputting input information and output information into an existing sentence comparison tool. The identification unit 103c then inputs the input information, the output information, and a constraint stipulating the output of sections where there are large semantic differences between the input and output information into the sentence generation model 300. The identification unit 103c may also identify recommended sections on the basis of sections where there are large semantic differences as obtained by inputting the constraint into the sentence generation model 300. Alternatively, the sections where there are large semantic differences may be obtained from a result of executing comparison processing by inputting the input information and the output information into a language model different from the sentence generation model 300.

The identification unit 103c may also compare inference results obtained by separately inputting the input information and the output information into an inference model that infers specific categories from sentences, and if the inference results differ, identify recommended sections on the basis of the sections that serve as the grounds of the inferences. In the case where the input information and the output information are structured sentences, the identification unit 103c may also identify recommended sections by comparison of sentences written in respective categories of the input information and the output information.

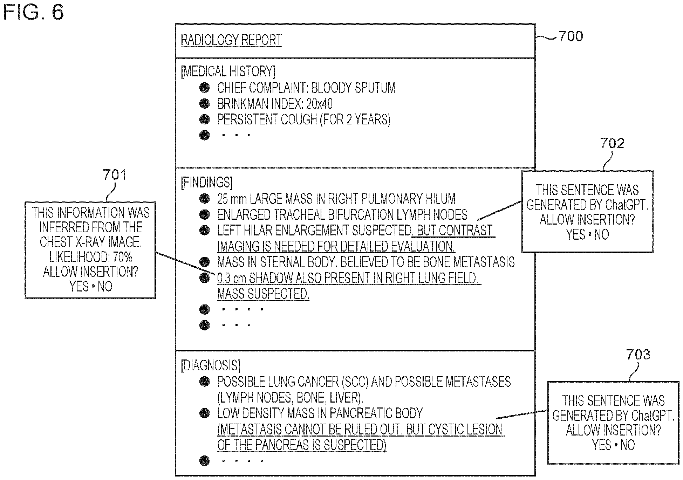

In step S104, the display control unit 103d causes the display unit, namely the display 105, to present a display 700 as illustrated in Fig. 6, in which the output information, namely the diagnostic report 600, is presented in correspondence with sections where user checking is recommended. For example, the display control unit 103d causes dialogs 701, 702, and 703 to be displayed with respect to sections where user checking is recommended. After the information processing, the information processing system 100 advances the process to the next step. The display control unit 103d causes display of an indication that there are sentences that have been corrected or added in the sections where user checking is recommended, and prompts the user to select whether or not to allow the insertion of such sentences. The above describes how, in step S102, the output information acquisition unit 103b causes the sentence generation model 300 to produce output information, that is, sentences inferred on the basis of the results of inferences made with respect to the chest X-ray image 500, and the likelihood of the inferences. In this case, for a section where a sentence has been added on the basis of an inference result, the display control unit 103d may also display an indication that the sentence has been added on the basis of the inference result, and the likelihood of the inference, as in the dialog 701, for example. Such indications may be displayed alongside the output information, namely the diagnostic report 400.

In step S105, the display control unit 103d accepts, in the dialogs 701, 702, and 703, the selection of whether or not to allow insertion of the sentences in the sections where checking is recommended. The user uses the input interface 104 or the like to make the selection. The process advances to step S106 to handle sentences in sections where the user has chosen not to allow sentence insertion. If all insertions are allowed, the input of the allowed sentences into the diagnostic report is finalized.

In step S106, the processing circuit 103 removes or corrects the sentences in sections where insertion is not allowed, reverting the text back to the sentences written in the input information, namely the diagnostic report 400. If new sentences were added by inference, the sentences are removed, and the diagnostic report is finalized.

By executing the above steps, the information processing system 100 can create reports using a sentence generation model while alleviating the user burden of checking.

Note that a diagnostic report that has been checked by the user may also be outputted as managed data to a data management device not illustrated, over the network 200 or via a communication cable or a communication circuit not illustrated.

### MODIFICATION 1 OF FIRST EMBODIMENT

In step S101, the input information acquisition unit 103a may acquire not only sentence information such as a diagnostic report, but also individual words, voice data, and the like. In cases where voice data is acquired, in step S102, the output information acquisition unit 103b inputs, into ChatGPT, sentence information converted from the voice data by analysis processing.

This can alleviate the user burden of inputting sentences.

### MODIFICATION 2 OF FIRST EMBODIMENT

In step S101, the input information acquisition unit 103a may acquire not only medical image data such as chest X-ray image data as an examination result, but also measured value data such as blood test or urine test results. A plurality of examination result data may also be acquired. In this case, in step S102, the output information acquisition unit 103b can acquire a diagnostic report containing, as output information, examination results outputted by the sentence generation model 300 and information inferred by the sentence generation model 300 on the basis of the examination result data.

For example, the output information acquisition unit 103b may also acquire the location and size of a mass detected from a chest X-ray image, C-reactive protein (CRP) level from a blood test, the presence or absence of inflammation inferred from the CRP value, and/or the like. The information acquired in this case is not information that was included in the input information, but rather is supplementary information added as a result of inferences made by the sentence generation model 300.

In step S104, the display control unit 103d displays each of the sections where checking is recommended, in correspondence with the corresponding examination results as the information source.

This makes it possible to supplement the diagnostic report on the basis of various examinations, thereby alleviating the user burden of reviewing the results of each examination and lowering the chance that the user will overlook information.

### MODIFICATION 3 OF FIRST EMBODIMENT

In step S103, the identification unit 103c acquires the diagnostic report 400, which is the input information that the user entered into the sentence generation model 300 such as ChatGPT, and the diagnostic report 600, which is the output information that the sentence generation model 300 outputted. Next, the identification unit 103c may identify sections where user checking is recommended by causing comparison processing to be performed by inputting the diagnostic report 400 and the diagnostic report 600 into a language model. Additionally, the identification unit 103c may input into the sentence generation model 300 a constraint stipulating that sections where there are large semantic differences are to be outputted in a distinguishable manner. In this case, the identification unit 103c may identify the outputted sections inferred to have large semantic differences as sections recommended for checking. A language model different from the sentence generation model 300 may also be used to infer which sections of the diagnostic report 400 and the diagnostic report 600 have large semantic differences. As another example, sections where the differences between the diagnostic report 400 and the diagnostic report 600 are determined not to be conjunctions or auxiliary words may be identified as sections where checking is recommended. With this modification, the identification unit 103c can identify sections inferred to have important differences between the diagnostic report 400 and the diagnostic report 600 as sections where user checking is recommended, thereby further alleviating the user burden of checking.

### MODIFICATION 4 OF FIRST EMBODIMENT

The processing circuit 103 may be further provided with a training unit. In step S104, the display control unit 103d extracts, from the sections displayed as sections recommended for checking, sentences that the user did not allow to be inserted or sentences chosen by prompting the user to decide whether a sentence is expressed in a desirable or undesirable way. The training unit may use the extracted sentences to perform additional training such as fine-tuning so that the sentence generation model 300 does not produce similar output in the future. In other words, the information processing system 100 is further provided with a training unit that performs additional training on the sentence generation model on the basis of user-provided input regarding the sections where user checking is recommended. Alternatively, in the case where the identification unit 103c is configured using an inference machine that infers the importance of differences between the input information and the output information, the training unit may train the inference machine by using sections extracted by the above processing as training data.

In step S104, the display control unit 103d may also prompt the user to enter appropriate sentences for the sections displayed as sections recommended for checking. In this case, the training unit uses an API to perform fine-tuning of the sentence generation model 300 and/or trains the above inference machine by using the appropriate sentences entered by the user as ground-truth output.

The information processing system 100 may also have a built-in function for performing fine-tuning of a sentence generation model 300 that is integrated with the information processing system 100.

With this arrangement, the sentence generation model becomes better at outputting appropriate sentences, thereby further alleviating the user burden of checking.

### MODIFICATION 5 OF FIRST EMBODIMENT

In step S104, the display control unit 103d may further prompt the user to select whether or not checking was unnecessary for sections displayed as sections recommended for checking. If checking was unnecessary, the identification unit 103c updates the identification criteria so that sections containing similar generated sentences will no longer be identified as sections where user checking is recommended. For example, the user may consider it unnecessary to check conjunctions such as "also" and "however", or auxiliary words such as "o" and "ga" in Japanese. In this case, the identification unit 103c calculates a degree of coincidence between sections where there are differences between the diagnostic results in the inputted diagnostic report 400 and the diagnostic report 600 outputted by ChatGPT, and sections for which checking is deemed unnecessary. The identification unit 103c calculates the degree of coincidence for "also", "however", "o", and "ga", and excludes sections with a high degree of coincidence from the sections recommended for checking.

The above describes processing for the case where, in step S103, the identification unit 103c uses a language model to determine sections where checking is recommended, in a similar manner to modification 3 of the first embodiment. In this case, in step S104, the identification unit 103c may also perform fine-tuning of the language model so that sections that the user has deemed unnecessary to check will no longer be determined as sections recommended for checking.

With this arrangement, sections containing minor differences between the input information, namely the diagnostic report 400, and the diagnostic report 600 are less likely to be recommended, thereby further alleviating the user burden of checking.

### MODIFICATION 6 OF FIRST EMBODIMENT

The diagnostic report 400 acquired by the input information acquisition unit 103a in step S101 and the diagnostic report 600 outputted by the sentence generation model 300 and acquired by the output information acquisition unit 103b in step S102 may also be structured reports. A structured report refers to a report in which sentences are written into each of one or more clinical categories. For example, the structured report may contain sentences written into each of the categories of medical history, findings, and diagnosis. In this case, in step S103, the identification unit 103c compares, for example, the sentences written in the findings category of the input information, namely the diagnostic report 400, with the sentences written in the findings category of the output information, namely the diagnostic report 600, outputted by the sentence generation model 300. If there are differences between the clinical categories, the identification unit 103c may identify sections containing the differences in the diagnostic report 600 outputted by the sentence generation model 300 as sections where user checking is recommended.

The sentences written in the categories to be compared between the reports may also be sentences written in categories such as medical history and diagnostic results, course of treatment and prognosis prediction, and similar cases. The identification unit 103c may also compare sentences written in multiple categories.

The user may also use the input interface 104 to preselect which categories are to be compared between the input information, namely the diagnostic report 400, and the output information, namely the diagnostic report 600, outputted by the sentence generation model 300. In this case, the identification unit 103c compares sentences only in the selected categories, and identifies sections containing differences as sections where user checking is recommended.

This facilitates recommendation to check sections containing changes pertaining to categories that the user wishes to check, thereby further alleviating the user burden of checking.

### MODIFICATION 7 OF FIRST EMBODIMENT

In step S103, the identification unit 103c may transmit a diagnostic report as input information to the sentence generation model 300, and further cause the sentence generation model 300 to infer the name of a disease. Specifically, the identification unit 103c is characterized as follows. The identification unit 103c acquires the input information, namely the diagnostic report 400, and the diagnostic report 600 outputted by the sentence generation model 300. The identification unit 103c causes the sentence generation model 300 to infer the name of a disease by separately inputting the diagnostic report 400 and the diagnostic report 600 into the sentence generation model 300. If the inferred names of a disease are different, the identification unit 103c uses the sentence generation model 300 to further acquire the sections in the diagnostic report 600 that serve as the grounds of the inference. The identification unit 103c may then identify the sections that serve as the grounds of the inference as sections where user checking is recommended. That is, the identification unit 103c acquires a first inference result inferred using an inference model that infers clinical categories from the diagnostic report constituting the input information. The identification unit 103c additionally acquires a second inference result inferred using the inference model from the diagnostic report constituting the output information. If the inference results are different, the identification unit 103c identifies sections of the output information that contribute to the second inference result as sections where user checking is recommended.

As another example, an inference model configured using a transformer architecture and trained on the task of inferring the names of diseases from diagnostic reports may be used to infer the name of a disease. When making an inference with respect to the diagnostic report 600, the identification unit 103c acquires sections with a high score calculated from values obtained from the attention layer of the inference model. The identification unit 103c may then identify the high-scoring sections as sections where user checking is recommended that contribute to the inference.

The category to be inferred from a diagnostic report may be whether or not re-examination is needed, course of treatment, prognosis prediction, or similar cases, for example. Multiple categories may also be inferred.

This facilitates recommendation to check sections containing changes pertaining to categories that the user wishes to check, thereby further alleviating the user burden of checking. Also, by making more accurate inferences using a model specialized in inferring categories that the user wishes to check, the probability of making incorrect checking recommendations can be lowered.

### SECOND EMBODIMENT

An information processing system 100 according to a second embodiment inputs the following into a sentence generation model 300 such as ChatGPT: input information, for example a diagnostic report, entered by a user such as a physician; and a constraint for controlling the output of the sentence generation model. The information processing system 100 identifies sections where user checking is recommended on the basis of output information based on the constraint, and causes the display to display the identified sections in correspondence with the information outputted by the sentence generation model. The constraint is a condition for causing the sentence generation model 300 to output newly created information in a distinguishable manner from the input information into the sentence generation model 300. In the present embodiment, the information processing system 100 includes an input information acquisition unit 103a that acquires input information for inputting into the sentence generation model and a constraint for causing the sentence generation model 300 to output newly created information in a distinguishable manner from the input information. Also included are an output information acquisition unit 103b that acquires output information resulting from inputting the input information into the sentence generation model 300, and an identification unit 103c that identifies sections of the output information where user checking is recommended. The information processing system 100 further includes a display control unit that causes a display to display the output information in correspondence with the sections where user checking is recommended. Note that the functional configuration of the information processing system 100 in the second embodiment is similar to the functional configuration of the information processing system in the first embodiment, and as such, the following description uses Fig. 1.

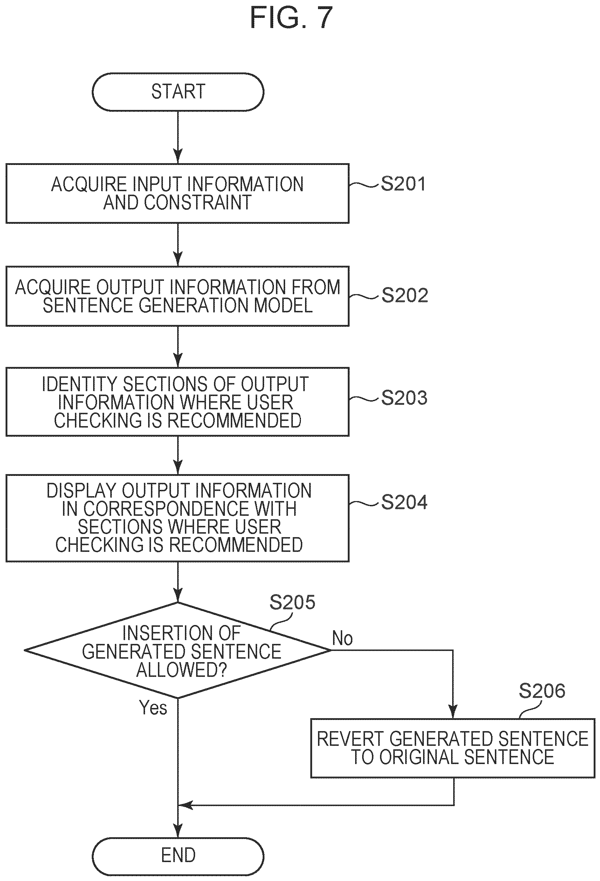

Fig. 7 is a flowchart illustrating the flow of an example of processing executed by the information processing system 100 according to the second embodiment. In the description of the present embodiment, it is assumed that execution of the processing illustrated in Fig. 7 is triggered when data to be processed is stored in the storage circuit 102 and the user operates the input interface 104 to give an instruction to start the processing. The data to be processed is text data of a diagnostic report 400 as illustrated in Fig. 3.
The following description follows the steps of the flowchart illustrated in Fig. 7. Note that the steps may be reordered insofar as the content is non-contradictory.

In step S201, the input information acquisition unit 103a acquires input information, namely the diagnostic report 400 to be processed, and a constraint for controlling the output of the sentence generation model 300.
For example, a prompt stating "Only expand upon the original text without changing it." is acquired as the constraint. The following describes a process flow under the assumption that the above constraint is acquired. Note that the content of the constraint is not limited to the above, and any kind of constraint may be acquired. For example, a constraint for outputting added sections in a distinguishable manner may also be entered as a prompt. For example, inputting the above constraint causes the sentence generation model 300 to output information with added sections in bold, a different character size, italics, and/or underlined.

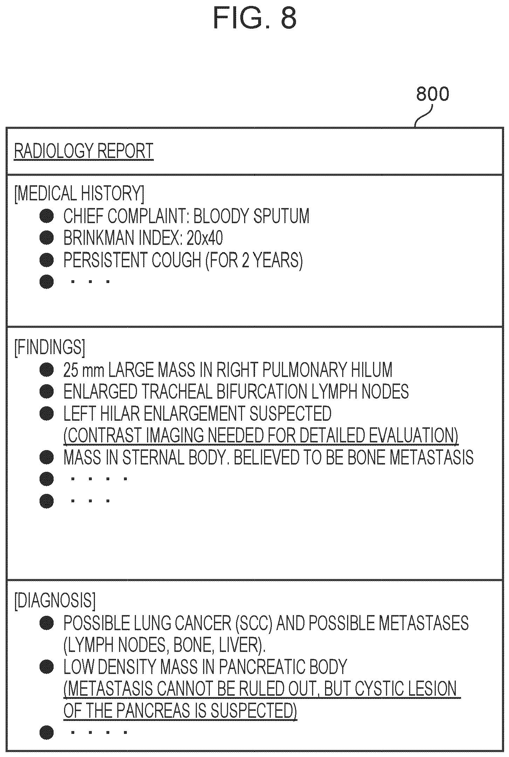

In step S202, the output information acquisition unit 103b acquires output information resulting from transmitting the diagnostic report 400 and the constraint to the sentence generation model 300. The output information is, for example, a diagnostic report 800 as illustrated in Fig. 8. For example, with respect to the sentence about left hilar enlargement written in the diagnostic report 400, a sentence such as "contrast imaging is needed for detailed evaluation" is added without modifying the text before or after, on the basis of the constraint. In addition, the added sentence is underlined so as to be distinguishable from the input information.

In step S203, the identification unit 103c, using the constraint as a basis, identifies sections of the output information, namely the diagnostic report 800, where user checking is recommended. For example, the identification unit 103c, using the constraint "Only expand upon the original text without changing it." as a basis, identifies differences between the input information, namely the diagnostic report 400, and the output information, namely the diagnostic report 800, as sections where user checking is recommended. In other words, the input information acquisition unit 103a is characterized by acquiring a constraint for controlling the sentence generation model 300 so as to only add newly created information. The identification unit 103c then identifies differences between the input information and the output information as the sections where user checking is recommended.

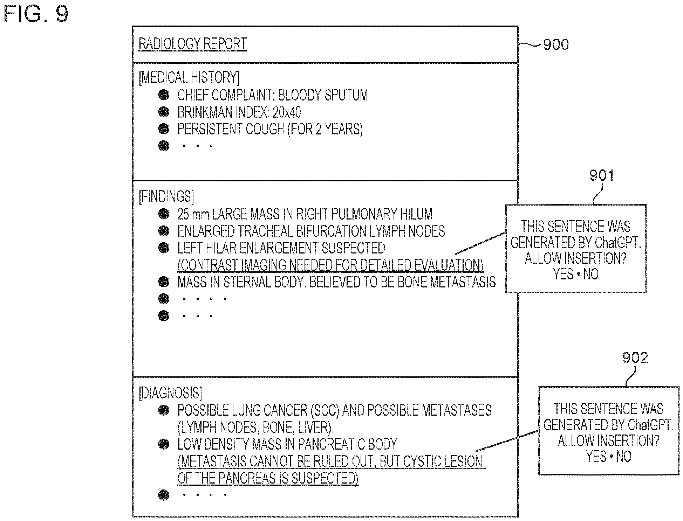

In step S204, the display control unit 103d causes the display unit, namely the display 105, to present a display 900 as illustrated in Fig. 9, in which the output information, namely the diagnostic report 800, is presented in correspondence with the sections where user checking is recommended as identified by the identification unit 103c. For example, as in the dialogs 901 and 902, the display control unit 103d causes display of an indication that there are sentences that have been corrected or added in the sections where user checking is recommended, and prompts the user to select whether or not to allow the insertion of such sentences.

In step S205, the display control unit 103d selects, in the dialogs 901 and 902, whether or not to allow insertion of the sentences in the sections where checking is recommended, according to a selection instruction given by the user using the input interface 104. The user uses the input interface 104 or the like to make the selection. The process advances to step S106 to handle sentences in sections where the user has chosen not to allow sentence insertion. If all insertions are allowed, the input of the allowed sentences into the diagnostic report is finalized.

In step S206, the processing circuit 103 removes or corrects the sentences in sections where insertion is not allowed, reverting the text back to the sentences written in the input information, namely the diagnostic report 400. If new sentences were added by inference, the sentences are removed, and the diagnostic report is finalized.

By executing the above steps, the information processing system 100 can create reports using a sentence generation model while alleviating the user burden of checking.

Note that a diagnostic report that has been checked by the user may also be outputted as managed data to a data management device not illustrated, over the network 200 or via a communication cable or a communication circuit not illustrated.

### MODIFICATION 1 OF SECOND EMBODIMENT

In step S201, the input information acquisition unit 103a may acquire a constraint for controlling the output of the sentence generation model 300 so as to output newly created information enclosed between leading and trailing **, which indicate bold type in Markdown syntax. In this case, in step S203, the identification unit 103c may use pattern matching to extract sections of the diagnostic report 800 outputted by the sentence generation model 300 which are enclosed between leading and trailing **, and identify the extracted sections as sections where user checking is recommended. The display control unit 103d may also display the sections where checking is recommended in bold type, in accordance with Markdown syntax.

The constraint may be anything whereby information newly created by the sentence generation model 300 is distinguishable from other sections. For example, newly created information may also be outputted so as to be enclosed between leading and trailing *, which indicate italic type in Markdown syntax, and may also be outputted so as to be enclosed between some other specific symbols.

With this arrangement, sections where checking is recommended can be displayed with formatting applied so that such sections are perceived more easily. Moreover, the processing by the identification unit 103c can be lightened, as the identification unit 103c can identify the sections where checking is recommended from the output information, namely the diagnostic report 800, outputted by the sentence generation model 300 without having to make a comparison with the input information.

### MODIFICATION 2 OF SECOND EMBODIMENT

In step S201, the input information acquisition unit 103a may acquire a constraint for controlling the output of the sentence generation model 300 so as not to change sections or categories containing diagnostic results in the input information, namely the diagnostic report 400. In other words, the input information acquisition unit 103a acquires a constraint stipulating that the sentence generation model 300 is to output information without changing sections containing clinical categories.

Also, depending on the constraint, the sections in which the output of the sentence generation model 300 is to be controlled may also be sections containing a course of treatment, prognosis prediction, similar cases, and/or the like, and may also be sections containing multiple categories each.

This allows for more consistent output of output information with respect to sections containing categories that the user does not want the sentence generation model 300 to change, thereby further alleviating the user burden of checking.

### MODIFICATION 3 OF SECOND EMBODIMENT

In step S201, the input information acquisition unit 103a causes the sentence generation model 300 to output diagnostic results inferred from the input information, namely the diagnostic report 400, and the sections that serve as the grounds of the inferences, each in a distinguishable manner from the diagnostic report 400. The input information acquisition unit 103a may acquire the acquired diagnostic results and the sections that serve as the grounds of the inferences, and cause a diagnostic report to be generated using the constraint for controlling the output of the sentence generation model 300 so as not to change the sections that serve as the grounds of the inferences.

The input information acquisition unit 103a may also separately input, into the sentence generation model 300, the input information, namely the diagnostic report 400, and the diagnostic report 800 outputted by the sentence generation model 300. The input information acquisition unit 103a may also acquire diagnostic results inferred from the diagnostic report and sections that serve as the grounds of the inferences, or in other words the sections that contribute to the inferences.

Furthermore, if the diagnostic results separately inferred by the sentence generation model 300 from the diagnostic report 400 and the diagnostic report 800 are different, the output information acquisition unit 103b executes the following processing. The output information acquisition unit 103b may input the diagnostic report 400 along with a constraint for controlling the output of the sentence generation model 300 so as not to change sections that served as the grounds of the inferences regarding the diagnostic report 400, and cause the diagnostic report 800 to be outputted again.

As another example, the input information acquisition unit 103a may infer diagnostic results by using an inference model configured using a transformer architecture and trained on the task of inferring diagnostic results from diagnostic reports. In this case, a constraint may be acquired to control the output of the sentence generation model 300 so as not to change sections with a high score calculated from values obtained from the attention layer of the inference model.

With this arrangement, clinically important sections that serve as the grounds of diagnosis are less likely to be changed, thereby further alleviating the user burden of checking.

### MODIFICATION 4 OF SECOND EMBODIMENT

In step S201, the input information acquisition unit 103a may further acquire medical image data and an inference model that infers the location of a mass from a medical image. The input information acquisition unit 103a may further acquire an inference result by applying the inference model to the medical image data, and establish a constraint for controlling the output of the sentence generation model 300 so as to produce output information accounting for the inference result. In this case, in step S204, the display control unit 103d may display the name of the inference model as the information source, in correspondence with sections where checking is recommended and/or in correspondence with the likelihood of the inference.

Moreover, the object of acquisition by the input information acquisition unit 103a and inference by the inference model may also be measured values from a blood test, a urine test, or the like, and may also be results from a plurality of tests.

The inference model may also infer a plurality of categories from a single test result, and may also make an inference based on a plurality of test results.

This enables the user to check the output information with consideration for the information-outputting entity and the likelihood, and thus allows for efficient report creation.

### MODIFICATION 5 OF SECOND EMBODIMENT

In step S201, the input information acquisition unit 103a may further acquire a past diagnostic report, and further acquire a constraint for controlling the output of the sentence generation model 300 so as to produce output accounting for the past diagnostic report. In this case, in step S202, the diagnostic report 800 acquired by the output information acquisition unit 103b contains inference results, for example, "The mass is larger than it was three years ago.", based on the past diagnostic report in comparison with the input information, namely the diagnostic report 400, and discrepancies inferred by comparison with the past diagnostic report, such as "The cough has persisted for three years, not two years."

Further, in step S204, the display control unit 103d may display the output information together with information pertaining to the input information, such as an indication that the output information was generated from the past diagnostic report and/or the date when the past diagnostic report was created. Alternatively, the display control unit 103d may display information pertaining to the input information in correspondence with sections where checking is recommended as identified by the identification unit 103c. Note that it is not strictly necessary to input a constraint, and a diagnostic report may also be obtained by inputting a past diagnostic report and the more recent diagnostic report 400 into a sentence generation model.

With this configuration, highly accurate output information that accounts for the information in the past report can be obtained, and by further displaying information pertaining to the input information serving as the input in correspondence with the output information, the user burden of checking can be alleviated.

### MODIFICATION 6 OF SECOND EMBODIMENT

In step S201, the input information acquisition unit 103a further acquires consultation guidelines as input information and acquires, as the constraint, information for controlling the output of the sentence generation model 300 so as to produce output accounting for the consultation guidelines. Specifically, in the diagnostic report 800 in step S202, information based on the consultation guidelines is added to the input information, namely the diagnostic report 400. The information to be added is sentence information such as "The lung carcinoma treatment newly written in the guidelines should also be considered in the future.", for example.

Also, in step S204, the display control unit 103d may display, as information pertaining to the input information, an indication that the output information was generated from the consultation guidelines and/or the date when the consultation guidelines were issued, and this information pertaining to the input information may also be displayed in correspondence with sections where checking is recommended.

With this arrangement, output information that accounts for user-created information other than diagnostic reports can be obtained, and by displaying the information serving as a source of input in correspondence with the output information, the user is able to carry out efficient checking with consideration for, among other things, the grounds of the sections to be checked.

### OTHER EMBODIMENTS

The present invention may also be carried out in various different forms other than the embodiments described above.

For example, the component elements of the devices illustrated in the drawings are functional and conceptual illustrations, and are not limited to being physically configured exactly as depicted in the drawings. In other words, the specific form in which the devices are separated or combined is not limited to the illustrations in the drawings, and all or part thereof may be functionally or physically separated or combined in freely chosen units according to various factors such as load and usage conditions. Furthermore, all or any part of the processing units to be achieved in each device may be achieved by a CPU and a program analytically executed by the CPU, or alternatively, achieved as hardware by wired logic.

Among the processes described in the embodiments, all or part of the processes described as being performed automatically can also be performed manually, and all or part of the processes described as being performed manually can also be performed automatically by known methods. Otherwise, information can be modified in any way unless specifically noted otherwise, such information including the processing procedures, control procedures, specific names, and various data and parameters indicated in the above document or in the drawings.

The method described in the embodiments can also be achieved by executing a prepared program on a computer such as a personal computer or a workstation. The program can be distributed over a network such as the Internet.
The control program can also be recorded to a non-transitory computer-readable recording medium, and can be executed by being read out from the recording medium by the computer. The non-transitory storage medium is a hard disk, floppy disk (FD), CD-ROM, magneto-optical (MO) disc, DVD, or the like.

According to at least one of the embodiments described above, it is possible to assist with the creation of reports using a sentence generation model while alleviating the user burden of checking.

Although several embodiments have been described, these embodiments are presented as examples only and are not intended to limit the scope of the invention to the specific embodiments disclosed. These embodiments can be implemented in various other forms, and various omissions, substitutions, modifications, and combinations of embodiments can be made without departing from the scope of the invention. These embodiments and modifications thereof are included within the scope of the claims and their equivalents, in a manner similar to the inclusion of these embodiments and modifications thereof within the scope of the invention. Each of the embodiments of the present disclosure can be implemented solely or as a combination of a plurality of the embodiments. Each feature or element of each embodiment of the present disclosure can be implemented as a combination of features of elements in a single embodiment. Each modification of one embodiment may be combined with one or more other modifications of the same embodiment. Each modification of one embodiment may be combined with one or more modification of the same embodiment or another embodiment wherein appropriate.

While the present invention has been described with reference to the above embodiments, it is to be understood that the invention is not limited solely to the disclosed embodiments. The scope of the following claims is to be accorded the broadest interpretation so as to encompass all such modifications and equivalent structures and functions.

## Claims

1. An information processing system comprising:
an input information acquisition unit (103a) configured to acquire input information for inputting into a sentence generation model;
an output information acquisition unit (103b) configured to acquire output information resulting from inputting the input information into the sentence generation model;
an identification unit (103c) configured to identify sections of the output information where user checking is recommended; and
a display control unit (103d) configured to cause a display to display the output information in correspondence with the sections where user checking is recommended.

2. The information processing system according to Claim 1, wherein the identification unit is configured to compare the input information with the output information to identify the sections where user checking is recommended.

3. The information processing system according to Claim 1 or Claim 2, wherein the input information acquisition unit is further configured to acquire a constraint for causing the sentence generation model to output newly created information in a distinguishable manner from the input information.

4. The information processing system according to any one of Claims 1 to 3, wherein the input information acquisition unit (103a) is configured to acquire information including sentences as the input information.

5. The information processing system according to any one of Claims 1 to 4, wherein the input information acquisition unit (103a) is configured to acquire input information including a constraint for inputting into the sentence generation model.

6. The information processing system according to any one of Claims 1 to 5, wherein the identification unit (103c) is configured to cause comparison processing to be performed by inputting the input information and the output information into the sentence generation model or a language model different from the sentence generation model.

7. The information processing system according to Claim 6, wherein the identification unit (103c) is configured to identify sections determined by the comparison processing to have large semantic differences between the input information and the output information as the sections where user checking is recommended.

8. The information processing system according to any one of Claims 1 to 7, wherein the display control unit (103d) is configured to cause the output information to be displayed in correspondence with an information source of the input information.

9. The information processing system according to any one of Claims 1 to 8, further comprising a training unit configured to perform additional training on the sentence generation model on the basis of user-provided input regarding the sections where user checking is recommended.

10. The information processing system according to any one of Claims 1 to 9, wherein the identification unit (103c) is configured to update criteria for identifying the sections where user checking is recommended on the basis of user-provided input regarding the sections where user checking is recommended.

11. The information processing system according to any one of Claims 1 to 10, wherein the input information comprises a diagnostic report concerning a subject.

12. The information processing system according to Claim 11, wherein the input information acquisition unit (103a) is further configured to acquire, as the input information, a past diagnostic report concerning the subject.

13. The information processing system according to Claim 11 or Claim 12, wherein the input information acquisition unit (103a) is further configured to acquire information on consultation guidelines as the input information.

14. The information processing system according to any one of Claims 11 to 13, wherein the identification unit (103c) is configured to compare the input information with the output information, and if there are differences between clinical categories, the identification unit (103c) is configured to identify sections containing the differences as the sections where user checking is recommended.

15. The information processing system according to any one of Claims 11 to 14, wherein if a first inference result inferred using an inference model configured to infer clinical categories from the diagnostic report constituting the input information and
a second inference result inferred using the inference model from a diagnostic report constituting the output information are different from one another,
the identification unit (103c) is configured to identify sections of the output information that contribute to the second inference result as the sections where user checking is recommended.

16. The information processing system according to any one of Claims 1 to 15, wherein the input information acquisition unit (103a) is further configured to acquire a constraint stipulating that the sentence generation model is to output information without changing sections containing clinical categories.

17. The information processing system according to any one of Claim 11 to 16, wherein the input information acquisition unit (103a) is further configured to acquire a constraint stipulating that an inference model that infers clinical categories from the diagnostic report is to produce output without changing sections that contribute to an inference result inferred from the input information.

18. The information processing system according to Claim 3 or any one of Claims 4 to 17 when dependent upon claim 3, wherein
the input information acquisition unit (103a) acquires the constraint for controlling the output of the sentence generation model so as to output newly created information enclosed between leading and trailing symbols which are distinguishable from the input information, and
the identification unit (103c) identifies sections of the output information enclosed between leading and trailing symbols as the sections where user checking is recommended.

19. An information processing method comprising:
acquiring input information for inputting into a sentence generation model;
acquiring output information resulting from inputting the input information into the sentence generation model;
identifying sections of the output information where user checking is recommended; and
causing a display to display the output information in correspondence with the sections where user checking is recommended.

20. A non-transitory storage medium storing a program comprising instructions for causing a computer to execute the information processing method according to Claim 19.
